(19) Europäisches Patentamt
European Patent Office
Office européen des brevets

(11) Publication number: **0 463 212 A1**

(12) **EUROPEAN PATENT APPLICATION**

(21) Application number: **90112254.9**

(22) Date of filing: **27.06.90**

(51) Int. Cl.5: **C07D 513/04**, A61K 31/425, //(C07D513/04,277:00,235:00)

(43) Date of publication of application: **02.01.92 Bulletin 92/01**

(84) Designated Contracting States: **BE CH DE ES FR GB IT LI NL SE**

(71) Applicant: **NIKKEN CHEMICALS CO., LTD.**
**4-14, Tsukiji 5-chome Chuo-ku**
**Tokyo(JP)**

(72) Inventor: **Seki, Taketsugu**
**11-2, Shima-cho**
**Omiya-shi, Saitama(JP)**
Inventor: **Tasaka, Shigeyuki**
**1143-9, Minaminakamaru**
**Omiya-shi, Saitama(JP)**
Inventor: **Hoshino, Ryuichi**
**1132-1, Oaza Shima**
**Omiya-shi, Saitama(JP)**

(74) Representative: **Vossius & Partner**
**Siebertstrasse 4 P.O. Box 86 07 67**
**W-8000 München 86(DE)**

(54) **Imidazo[2,1-b]thiazole compound anti-ulcer agent containing the same.**

(57) The present invention relates to imidazo[2,1-b]thiazole compounds represented by formula (I) and pharmacologically acceptable salts thereof:

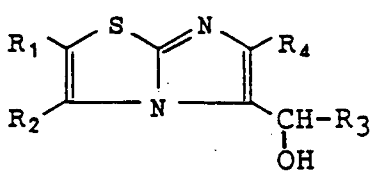

(I)

wherein $R_1$ and $R_2$ may be the same or different groups and each represents a hydrogen atom or a lower alkyl group having 1 to 6 carbon atoms; $R_3$ represents a hydrogen atom, a lower alkyl group having 1 to 6 carbon atoms or a phenyl group which may be optionally substituted by methyl group, methoxy group or halogen; and $R_4$ represents a hydrogen atom or a lower alkyl group having 1 to 3 carbon atoms, with the proviso that when $R_3$ represents a hydrogen atom, at least one of $R_1$ and $R_2$ represents a lower alkyl group having 1 to 6 carbon atoms, and antiulcer agents comprising said compounds or salts thereof as the active ingredient.

EP 0 463 212 A1

The present invention relates to imidazo[2,1-b]thiazole compounds or pharmacologically acceptable salts thereof and antiulcer agent containing the same as the active ingredient.

Heretofore, many imidazo[2,1-b]thiazole compounds have been isolated and known. With regard to the pharmacological activity of these compounds, it has been disclosed that they have analgesic and antiphlogistic activity (see, JP-B-59-40835 (the term "JP-B" as used herein means an "examined Japanese patent publication") and JP-B-59-53278), anti-thrombotic activity (see, JP-A-56-115780 (the term "JP-A" as used herein means an "unexamined published Japanese patent application"), treatment activity to diabetes (see, JP-A-52-83586), antihypertensive and diuretic activity (see, JP-A-57-21389, corresponding to U.S. Patent 4,444,770) and antitumor activity (see, A. Andreani et al, Il Farmaco Ed. Sc., vol. 35, fasc. 11, 896-901). However, neither specific disclosure nor even suggestion has been found on medical application of the compounds as antiulcer agents.

In general, it is considered that peptic ulcers would be caused when an imbalance occurred between attack factors such as gastric juice, pepsin, etc. and mucus, bicarbonate ion secretion phase, blood flow or the like. Their development is specific to stomach and duodenum. Medical drug therapy for these peptic ulcers has been shifted from therapy laying stress on antiacidic agents or anticholinergic agents to therapy attaching importance to an antagonists for histamine $H_2$ receptor showing a strong acid secretion inhibitory activity by blocking gastric wall cell receptor. However, it is reported in Marks, I. N. et al., "Ulcer healing and relapse rate after initial treatment with cimetidine or sucralfate", J. Clin. Gastroent., 3 (Suppl. 2), 163-165 (1981) and Martin, D. F. et al., "Difference in relapse rates of duodenal ulcer after healing with cimetidine or tripotassium dictrato bismuthate", Lancet, I, 7-10 (1981) that when administration of the histamine $H_2$ receptor antagonists was discontinued, relapse of ulcer was noted with high frequency.

In recent years, based on a new finding in acid secretion mechanism in gastric wall cells and mucus protecting mechanism, antiulcer agents which inhibit $[H^+-K^+]$ adenosine triphosphatase (ATPase) participating in the final stage during the course of acid secretion in wall cells and which prevent secretion of gastric juice have been proposed, for example, in JP-B-60-34956 (corresponding to U.S. Patent 4,255,431).

The present inventors have synthesized many analogous compounds using known imidazole or thiazole compounds as starting materials and made extensive investigations on these compounds. As a result, it has been found that while imidazo[2,1-b]thiazole compounds according to the present invention have a chemical structure different from known drugs showing an antiulcer activity, the compounds exhibit an excellent antiulcer activity. The present invention has been to be achieved based on the findings.

An object of the present invention is to provide imidazo[2,1-b]thiazole compounds represented by the following formula (I) and pharmacologically acceptable salts thereof.

Another object of the present invention is to provide antiulcer agents comprising the imidazo[2,1-b]-thiazole compounds of formula (I) or pharmacologically acceptable salts thereof as active ingredient.

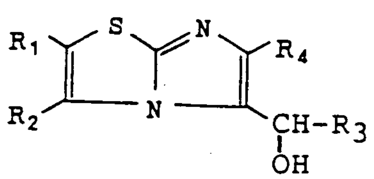

(I)

wherein $R_1$ and $R_2$ may be the same or different groups and each represents a hydrogen atom or a lower alkyl group having 1 to 6 carbon atoms; $R_3$ represents a hydrogen atom, a lower alkyl group having 1 to 6 carbon atoms or a phenyl group which may be optionally substituted by methyl group, methoxy group or halogen; and $R_4$ represents a hydrogen atom or a lower alkyl group having 1 to 3 carbon atoms, with the provision that when $R_3$ is a hydrogen atom, at least one of $R_1$ and $R_2$ is a lower alkyl group having 1 to 6 carbon atoms.

The imidazo[2,1-b]thiazole compounds represented by the formula (I) according to the present invention are all novel compounds. The compounds and pharmacologically acceptable salts thereof are useful in the medical field as antiulcer agents due to gastric juice secretion inhibiting activity.

Now, the compounds represented by the formula (I) will be illustrated in more detail below.

$R_1$ and $R_2$ may be the same or different groups and each is a hydrogen atom or a lower alkyl group having 1 to 6 carbon atoms such as methyl group, ethyl group, isopropyl group, butyl group, etc. and preferably each is a hydrogen atom or a lower alkyl group having 1 to 4 carbon atoms. $R_3$ is a hydrogen atom, a lower alkyl group having 1 to 6 carbon atoms, a phenyl group or a substituted phenyl group having 1 to 3 substituent(s) such as methyl group, methoxy group, halogen or the like, preferably $R_3$ is a hydrogen atom, a lower alkyl group having 1 to 4 carbon atoms or an unsubstituted phenyl group. $R_4$ is a hydrogen

atom or a lower alkyl group having 1 to 3 carbon atoms, preferably $R_4$ is a methyl group. When $R_3$ is a hydrogen atom, at least one of $R_1$ and $R_2$ is a lower alkyl group having 1 to 6 carbon atoms, preferably $R_2$ is a lower alkyl group having 1 to 6 carbon atoms.

Preferred examples of the compounds include 3,6-dimethyl-5-hydroxymethylimidazo[2,1-b]thiazole (the compound of Example 1), 5-(hydroxy-phenyl)methyl-6-methylimidazo[2,1-b]thiazole(the compound of Example 3) and 5-(1-hydroxybutyl)-6-methylimidazo[2,1-b]thiazole (the compound of Example 4).

The imidazo[2,1-b]thiazole compounds represented by the formula (I) according to the present invention can be produced in the following manner.

Process I:

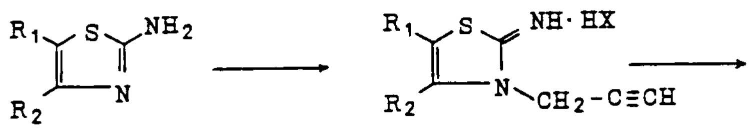

(II) (II-a)

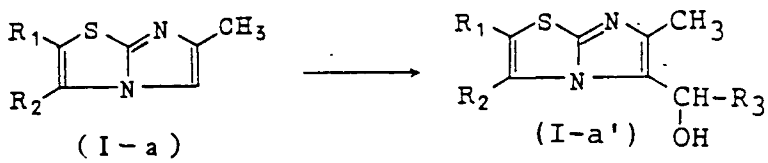

(I-a) (I-a')

wherein $R_1$, $R_2$ and $R_3$ are as defined above and X represents a halogen.

Process II:

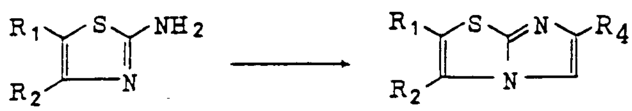

(II) (I-b)

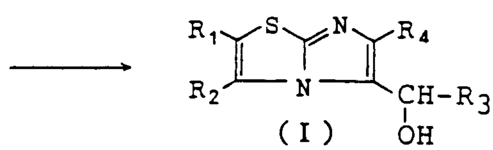

(I)

wherein $R_1$, $R_2$, $R_3$ and $R_4$ are as defined above.

Namely, the imidazo[2,1-b]thiazole compounds represented by the formula (I) (including the compounds represented by the formula (I-a')) can be produced by using 2-aminothiazole compounds represented by the formula (II).

Now, these processes will be illustrated in more detail below.

\1\ Process I wherein a 2-aminothiazole compound (II) is reacted with a propargyl halide in a solvent such as an alcohol (e.g., ethanol, n-butanol, etc.), tetrahydrofurane, or methyl ethyl ketone under reflux or at room temperature for 2 hours to 4 days and the resulting 2-imino-3-propargylthiazole compound (II-a) is cyclized in the presence of a condensing agent such as sodium hydroxide or sodium alkoxide in the above-described alcohol,

\2\ Process II wherein a 2-aminothiazole compound (II) is reacted with a halogenated ketone compound such as chloroacetaldehyde or bromoacetone in an solvent such as ethanol n-butanol methyl ethyl ketone or tetrahydrofurane at room temperature to 120 °C for 2 hours to 4 days to effect cyclization.

A suitable process can be chosen among these processes and applied to the production of the

compounds of the present invention.

The compounds represented by the formula (II) which are used as the starting materials in these processes are readily available or can be readily synthesized by those skilled in the art, if necessary. For example, these starting materials can be synthesized according to the methods described in Journal of the American Chemical Society, Vol. 68, P.457 (1946) and ibid., Vol.71, P.4007 (1949).

Further, when a $-CH(OH)-R_3$ group is introduced into the 5-position of the compound represented by the formula (I-a) or (I-b), the substitution reaction can be carried out according to known reactions such as Vilsmeier reaction, Grignard reaction or a reduction reaction to prepare a desired compound.

The imidazo[2,1-b]thiazole compounds represented by the formula (I) (including the compounds represented by the formula (I-a')) which are synthesized by the processes described above can be separated from the reaction mixture and purified by conventional methods such as extraction with a solvent, chromatography, crystallization, etc.

Further, the compounds represented by the formula (I) which are produced by the processes described above can be converted into pharmacologically acceptable salts thereof, if necessary and desired. Examples of acid addition salts of these compounds are addition salts with inorganic acids such as hydrochloric acid, sulfuric acid, nitric acid, hydrobromic acid, etc.; addition salts with organic acids such as oxalic acid, citric acid, methanesulfonic acid, p-toluenesulfonic acid, etc., preferably a hydrochloride thereof.

Accordingly, when the imidazo[2,1-b]thiazole compounds represented by the formula (I) are converted into pharmacologically acceptable salts thereof, for example, into acid addition salts, the compounds of the present invention are reacted with stoichiometrical amounts of acids in a suitable solvent such as ethanol or acetone to produce the salts.

The compounds produced in the present invention may involve optically active isomers such as dextrorotary and levorotary isomers and a mixture thereof, etc. All of these compounds are included within the scope of the present invention.

The compounds of the present invention possesses interesting pharmacological properties and are useful as antiulcer agents due to acid secretion inhibiting activity.

In the case of using the compounds in accordance with the present invention as antiulcer agents, the agents can be administered by appropriate mode such as oral or parenteral route. Examples of oral administration include tablets, granules, capsules, pills, powders, etc. Examples of parenteral administration include an injection, suppository, liquid, etc. In making these compositions for medical administration, the compound (and its salts) of the present invention can be formulated into medical preparation in a conventional manner. For example, in the case of oral compositions, the compound may be formulated into desired administration forms, using excipients such as lactose, glucose, corn starch, sucrose, etc.; disintegrators such as carboxymethyl cellulose calcium, hydroxypropyl cellulose, etc.; lubricants such as calcium stearate, magnesium stearate, talc, polyethylene glycol, hardened oil, etc.; binders such as hydroxypropylcellulose, hydroxypropylmethylcellulose, carboxymethylcellulose, polyvinyl alcohol, gelatin, gum arabic, etc.; wetting agents such as glycerine, ethylene glycol, etc.; and, if desired, surface active agents, corrigents, etc.

On the other hand, in the case of parenteral compositions, the compound may be formulated into parenteral preparations using diluents such as water, ethanol, glycerine, propylene glycol, polyethylene glycol, agar, tragacanth gum, etc., if desired, further using dissolution aids, buffers, preservatives, fragrances, coloring matters, etc.

In the administration of the compounds (or its salts) of the present invention as antiulcer agents, they may be given as a unit dose (being converted into the weight of the compounds of formula (I)) in a range of 50 to 800 mg, preferably 100 to 400 mg per adult per day in the case of oral administration and in the case of parenteral administration, in a range of 10 to 300 mg, preferably 30 to 200 mg, per adult per day. Desired therapeutic effects can be expected through administration by dividing the dose into one to 3 times per day.

The present invention is now illustrated in greater detail by reference to the following examples which, however, are not to be construed as limiting the invention in any way.

## EXAMPLE 1

### Synthesis of 3,6-dimethyl-5-hydroxymethylimidazo[2,1-b]thiazole

3,6-Dimethyl-5-formylimidazo[2,1-b]thiazole (1.8g) was suspended in 40 mℓ of ethanol. To the suspension was added 0.18g of sodium boron hydride and the mixture was warmed at 50°C for 30 minutes. The reaction mixture was evaporated to dryness, and the residue was washed with water to give 1.78g of the title compound.

Melting point: 169.0 - 171.0 °C
NMR δ (DMSO - $d_6$)
2.0 - 2.7 (b, 1H, OH)
2.22 (s, 3H, $CH_3$)
2.61 (d, 3H, $CH_3$)
4.73 (s, 2H, $CH_2$)
6.27 (q, 1H, skeleton)

EXAMPLE 2

Synthesis of 2-butyl-3,6-dimethyl-5-hydroxymethylimidazo[2,1-b]thiazole

In 40 mℓ of ethanol was suspended 1.80g of 2-butyl-3,6-dimethyl-5-formylimidazo[2,1-b]thiazole. To the suspension was added 0.18g of sodium born hydride and the mixture was warmed at 50 °C for 30 minutes. The reaction mixture was evaporated to dryness. The residue was washed with water to give 1.71g of the title compound.
Melting point: 173.0 - 174.0 °C
NMR δ (DMSO - $d_6$)
1.91 (t, 3H, $CH_2CH_3$)
2.20 (s, 3H, $CH_3$)
2.51 (s, 3H, $CH_3$)
4.57 (d, 2H, $CH_2OH$)
5.02 (t, 1H, OH)

EXAMPLES 3 to 12

5-Formyl-6-methylimidazo[2,1-b]thiazole,3,6-dimethyl-5-formylimidazo[2,1-b]thiazole or 2-butyl-3,6-dimethyl-5-formyl-imidazo[2,1-b]thiazole was dissolved (20 m mol) in 30 mℓ of tetrahydrofuran. Under cooling, the corresponding Grignard reagent was added to the solution. The mixture was stirred for 30 minutes and then allowed to stand at room temperature for 3 hours or heated at 50 °C for 30 minutes. After cooling, the reaction mixture was neutralized with hydrochloric acid and extracted with 30 to 50 mℓ of ethyl acetate repeatedly twice to 3 times. The desired product was obtained from the ethyl acetate phase.

The compound synthesized by each of these examples is shown in Table 1.

Table 1 (Examples 3 to 12)

| Ex. | Product | Starting material | Melting point (°C) | NMR | Yield (%) |
|---|---|---|---|---|---|
| 3 | 5-(hydroxy-phenyl)-methyl-6-methylimidazo[2,1-b]thiazole | 5-formyl-6-methyl-imidazo[2,1-b]-thiazole | 148.5 - 149.0 | δ (DMSO-$d_6$)<br>2.24 (s, 3H, $CH_3$)<br>6.02 (s, 1H, CH)<br>7.01 (d, 1H, skeleton)<br>7.42 (d, 1H, skeleton) | 93.8 |
| 4 | 5-(1-hydroxybutyl)-6-methylimidazo[2,1-b]-thiazole | 5-formyl-6-methyl-imidazo[2,1-b]-thiazole | 139.5 - 140.5 | δ ($CDCl_3$)<br>1.90 (t, 3H, $(CH_2)_2\underline{CH_3}$)<br>2.13 (s, 3H, $CH_3$)<br>4.87 (t, 1H, CH)<br>6.65 (d, 1H, skeleton)<br>7.53 (d, 1H, skeleton) | 79.4 |
| 5 | 5-(1-hydroxyethyl)-6-methylimidazo[2,1-b]-thiazole | 5-formyl-6-methyl-imidazo[2,1-b]-thiazole | 151.5 - 152.2 | δ ($CDCl_3$)<br>2.12 (s, 3H, $CH_3$)<br>2.52 (d, 3H, $CH_3$)<br>5.07 (q, 1H, CH)<br>6.65 (d, 1H, skeleton)<br>7.55 (d, 1H, skeleton) | 80.3 |

Table 1 (Cont'd)

| Ex. | Product | Starting material | Melting point (°C) | NMR | Yield (%) |
|---|---|---|---|---|---|
| 6 | 2-butyl-3,6-dimethyl-5-(1-hydroxybutyl)-imidazo[2,1-b]thiazole | 2-butyl-3,6-dimethyl-5-formyl-imidazo[2,1-b]-thiazole | 81.0 - 82.0 | δ ($CDCl_3$)<br>1.92 (t, 3H, $CH_2\underline{CH_3}$)<br>2.23 (s, 3H, $CH_3$)<br>2.50 (s, 3H, $CH_3$)<br>2.60 (t, 3H, $CH_3$)<br>4.99 (t, 1H, CH) | 86.3 |
| 7 | 2-butyl-3,6-dimethyl-5-(hydroxy-phenyl)-methylimidazo[2,1-b]-thiazole | 2-butyl-3,6-dimethyl-5-formylimidazo-[2,1-b]thiazole | 134.0 - 135.0 | δ ($CDCl_3$)<br>1.88 (t, 3H, $CH_2\underline{CH_3}$)<br>1.97 (s, 3H, $CH_3$)<br>2.01 (s, 3H, $CH_3$)<br>2.52 (t, 3H, $CH_2$)<br>6.13 (s, 1H, CH) | 81.6 |
| 8 | 3,6-dimethyl-5-(hydroxy-phenyl)-methylimidazo[2,1-b]-thiazole | 3,6-dimethyl-5-formylimidazo-[2,1-b]thiazole | 170.0 - 173.0 | δ (DMSO-$d_6$)<br>2.11 (t, 3H, $CH_3$)<br>2.12 (d, 3H, $CH_3$)<br>6.13 (s, 2H, CH-OH)<br>6.59 (q, 1H, skeleton)<br>7.25 (m, 5H, benzene) | 87.5 |

Table 1 (Cont'd)

| Ex. | Product | Starting material | Melting point (°C) | NMR | Yield (%) |
|---|---|---|---|---|---|
| 9 | 5-{hydroxy-(4-methylphenyl)}methyl-6-methylimidazo[2,1-b]thiazole | 5-formyl-6-methylimidazo[2,1-b]thiazole | 178 - 180 | δ ($CDCl_3$)<br>2.23 (s, 3H, $CH_3$)<br>2.35 (s, 3H, $CH_3$)<br>6.11 (s, 1H, CH)<br>6.59 (d, 1H, skeleton)<br>7.05-7.35 (m, 6H, skelton, benzene, OH) | 79.0 |
| 10 | 5-{hydroxy-(3,4-dimethoxyphenyl)}-methyl-6-methyl-imidazo[2,1-b]thiazole | 5-formyl-6-methylimidazo[2,1-b]thiazole | 168 - 170 | δ ($CDCl_3$)<br>2.15 (s, 3H, $CH_3$)<br>3.85 (s, 3H, $OCH_3$)<br>3.87 (s, 3H, $OCH_3$)<br>6.07 (s, 1H, CH)<br>6.59 (d, 1H, skeleton)<br>6.8-7.1 (m, 4H, benzene, OH)<br>7.19 (d, 1H, skeleton) | 35.0 |

Table 1 (Cont'd)

| Ex. | Product | Starting material | Melting point (°C) | NMR | Yield (%) |
|---|---|---|---|---|---|
| 11 | 5-{hydroxy-(4-fluorophenyl)}methyl-6-methylimidazo[2,1-b]thiazole | 5-formyl-6-methylimidazo[2,1-b]thiazole | 162 - 163 | δ ($CDCl_3$)<br>2.18 (s, 3H, $CH_3$)<br>6.08 (s, 1H, CH)<br>6.60 (d, 1H, skeleton)<br>6.98-7.04 (m, 2H, benzene)<br>7.12 (d, 1H, skeleton)<br>7.31-7.35 (m, 2H, benzene) | 73.2 |
| 12 | 3,6-dimethyl-5-{hydroxy-(4-fluorophenyl)}-methylimidazo[2,1-b]-thiazole | 3,6-dimethyl-5-formylimidazo[2,1-b]thiazole | 201 - 202 | δ ($CDCl_3$)<br>2.07 (d, 3H, $CH_3$)<br>2.10 (s, 3H, $CH_3$)<br>6.18 (d, 1H, CH)<br>6.97-7.03 (m, 2H, benzene)<br>7.23-7.28 (m, 3H, benzene) | 60 |

EXAMPLE 13

Synthesis of 5-hydroxymethylimidazo[2,1-b]thiazole

In 40 mℓ of ethanol was suspended 1.8g of 5-formylimidazo[2,1-b]thiazole. To the suspension, there

was added 1.89g of sodium borohydride. The mixture was warmed at 50 °C for 30 minutes. The reaction mixture was then evaporated to dryness, and the residue was washed with water to give the title compound in a yield of 99.2%.
Melting point: 117 °C (decomposed)
NMR δ (DMSO-$d_6$)
4.60(s, 2H, $CH_2$), 7.06(d, 1H, skeleton),
7.20(q, 1H, skeleton) 7.77(d, 1H, skeleton)

EXAMPLE 14

| (Preparation of tablets) | |
|---|---|
| Compound (Example 3) of the invention | 250g |
| Lactose | 620g |
| Corn starch | 400g |
| Hydroxypropyl cellulose | 20g |
| Magnesium stearate | 10g |

After the compound of the present invention, lactose and corn starch described above were thoroughly mixed to become homogeneous, 5 w/v% ethanolic solution of hydroxypropyl cellulose was added to the mixture. The mixture was kneaded to prepare into granules. After graining through a sieve of 16 mesh, tableting was performed in a conventional manner to prepare tablets having a weight of 130 mg and a diameter of 7 mm and containing 25 mg of the compound, per tablet.

EXAMPLE 15

| (Preparation of capsules) | |
|---|---|
| Compound (Example 4) of the invention | 250g |
| Lactose | 620g |
| Avicel | 620g |
| Magnesium stearate | 10g |

After the compound of the present invention, lactose, Avicel and stearic magnesium were thoroughly mixed to become homogeneous, the mixture was filled up in No. 3 capsules to prepare capsules having a weight of the content of 150 mg and containing 25 mg of the compound, per capsule.

EXAMPLE 16

| (Preparation of granules) | |
|---|---|
| Compound (Example 1) of the invention | 100g |
| White sugar powder | 300g |
| Lactose | 200g |
| Corn starch | 390g |
| Hydroxypropyl cellulose | 10g |

After the compound of the present invention, white sugar powder, lactose and corn starch described above were thoroughly mixed to become homogeneous, 20% hydrated ethanolic solution of 5% hydroxypropyl cellulose was added to the mixture. The mixture was kneaded and then grained through a sieve of 24 mesh. After drying, the grains were passed through a sieve of 15 mesh for further graining. Per 1 g of the granules, 100 mg of the compound was contained.

EXAMPLE 17

| (Preparation of granulates) | |
|---|---|
| Compound (Example 4) of the invention | 50g |
| Lactose | 400g |
| Corn starch | 300g |
| Avicel | 220g |
| Polyvinyl pyrrolidone | 30g |

After the compound of the present invention, lactose, corn starch and Avicel described above were thoroughly mixed to become homogeneous, 10 w/v% methanolic solution of polyvinylpyrrolidone was added to the mixture. After kneading the mixture, the mixture was ground and grained with a grinder. After drying, the mixture was passed through a sieve of 20 mesh for granulation. Per 1g of the granulates, 50 mg of the compound was contained.

The present invention is further illustrated in greater detail by reference to the following test examples which demonstrate the excellent pharmacological characteristic of the compounds of the present invention.

EXPERIMENT 1

Antiulcer activity against aspirin-induced ulcer model

A suspension of each test compound in 5% gum arabic aqueous solution was orally given to Wistar strain male rats (at the age of 7 to 8 weeks, weighing 180 to 227 g; 7-8 rats in the group administered with the test compound; 14-16 rats in the control group) fasted for 24 hours at a dose of 100 mg of the test compound/5 ml/kg. Thirty minutes after administration, aspirin was further orally administered at a dose of 200 mg/kg. Seven hours after administration of aspirin, stomach was excised under ethereal anesthesia and spotted and linear erosion and a long diameter of ulcer were microscopically measured to determine an ulcer index and an inhibition rate.

In the control group, 5% gum arabic aqueous solution was orally administered at a dose of 5 ml/kg.

The results are shown in Table 2.

Table 2

| Test compound | Ulcer index (mm) | Inhibition rate (%) |
|---|---|---|
| Control | 70.9 ± 6.3 | -- |
| Ex. 1 | 38.6 ± 7.9** | 45.6 |
| Ex. 2 | 52.7 ± 7.8 | 25.7 |
| Ex. 5 | 29.6 ± 6.8** | 58.3 |
| Ex. 6 | 37.5 ± 7.4** | 47.1 |
| Ex. 7 | 65.1 ± 8.5 | 8.2 |
| Control | 57.4 ± 5.3 | -- |
| Ex. 3 | 31.7 ± 5.9** | 44.8 |
| Control | 66.8 ± 6.5 | -- |
| Ex. 4 | 41.3 ± 3.8** | 38.2 |

** $P < 0.01$

EXPERIMENT 2

Antiulcer activity against water-immersion stress ulcer model

A suspension of each test compound in 5% gum arabic aqueous solution was orally given to Wistar strain male rats (at the age of 7 to 8 weeks, weighing 191 to 238 g; 7-8 rats in the group administered with the test compound; 14-16 rats in the control group) fasted for 24 hours at a dose of 100 mg of the test compound/5 ml/kg. Thirty minutes after administration, the animal was immersed up to the xiphisternum in water bath at 22 ± 1 °C using a stress cage made by Tokyo University, Faculty of Pharmacology to apply a stress load for 6 hours. After applying the stress load, the rats had the cervical vertebra dislocated to death. Then, the stomach was excised and, spotted and linear erosion and a long diameter of ulcer were microscopically measured to determine an ulcer index and an inhibition rate.

In the control group, 5% gum arabic aqueous solution was orally administered at a dose of 5 ml/kg.

The results are shown in Table 3.

Table 3

| Test compound | Ulcer index (mm) | Inhibition rate (%) |
|---|---|---|
| Control | 23.7 ± 3.8 | -- |
| Ex. 1 | 2.6 ± 0.9** | 89.0 |
| Ex. 2 | 13.3 ± 3.9 | 43.9 |
| Ex. 5 | 19.5 ± 5.8 | 17.7 |
| Ex. 6 | 18.8 ± 4.7 | 20.7 |
| Ex. 7 | 25.5 ± 5.5 | -- |
| Control | 21.5 ± 2.7 | -- |
| Ex. 3 | 3.6 ± 2.0*** | 83.3 |
| Control | 23.3 ± 3.1 | -- |
| Ex. 4 | 7.4 ± 1.5*** | 68.2 |
| Control | 16.3 ± 4.6 | -- |
| Ex. 8 | 2.8 ± 0.5* | 82.0 |

* $P < 0.05$ ** $P < 0.01$
*** $P < 0.001$

EXPERIMENT 3

Antiulcer activity against ethanol-induced ulcer model

A suspension of each test compound in 5% gum arabic aqueous solution was orally given to Wistar strain male rats (at the age of 7 to 8 weeks, weighing 180 to 250 g; 5-9 rats in the group administered with the test compound; 4-9 rats in the control group) fasted for 24 hours at a dose of 100 mg of the test compound/5 ml/kg. Thirty minutes after administration, absolute ethanol was further orally administered at a dose of 5 mℓ/kg. One hour after administration of absolute ethanol, the stomach was excised under ethereal anesthesia and the area of an ulcerated portion ($mm^2$) (using microscope) or the proportion (%) thereof to all area of gastric wall (by computer image analysis) were measured to determine an ulcer index and an inhibition rate.

In the control group, 5% gum arabic aqueous solution was orally administered at a dose of 5 ml/kg.

The results are shown in Table 4.

Table 4

| Test compound | Ulcer index ($mm^2$) | Inhibition rate (%) |
|---|---|---|
| Control | 158.9 ± 26.2 | -- |
| Ex. 4 | 9.2 ± 3.1** | 94.2 |
| Control | 86.0 ± 18.2 | -- |
| Ex. 8 | 15.3 ± 8.5** | 82.2 |

| Test compound | Ulcer index (%) | Inhibition rate (%) |
|---|---|---|
| Control | 16.9 ± 4.0 | -- |
| Ex. 9 | 6.8 ± 1.5* | 59.8 |
| Ex. 10 | 2.7 ± 0.9** | 84.0 |
| Ex. 11 | 1.2 ± 0.5** | 92.9 |
| Ex. 12 | 6.3 ± 1.3* | 62.7 |

* $P<0.05$, ** $P<0.01$

EXPERIMENT 4

Gastric juice secretion inhibition activity

A suspension of each test compound in 5% gum arabic aqueous solution was orally given to Wistar strain male rats (at the age of 7 to 8 weeks, weighing 192 to 241 g; 7-9 rats in the group administered with the test compound; 15 rats in the control group) fasted for 24 hours at a dose of 100 mg of the test compound/5 ml/kg. Thirty minutes after administration (the compounds of Examples 3 and 4) or 60 minutes after administration (the compounds of Examples 1, 2, 5, 6 and 7), pyloric ligature was made under ethereal anesthesia. Four hours after, the stomach was excised under ethereal anesthesia to collect gastric juice. The collected gastric juice was centrifuged (2500 rpm) at 4°C for 10 minutes. After the supernatant was collected, an amount of the gastric juice and its pH were measured using a pH meter. Furthermore, an amount of free hydrochloric acid and the total acidity were measured with Töpfer reagent and phenolphthalein reagent, respectively. Using a part of the gastric juice, pepsin output in the gastric juice was measured using casein as substrate in a manner similar to the method of Anson et al.

In the control group, 5% gum arabic aqueous solution was orally administered at a dose of 5 ml/kg.

The results are shown in Tables 5 and 6.

Table 5

| Test compound | Gastric juice (mℓ/100g) | Gastric juice (pH) | Free HCℓ (mcEq/100g) | Total acidity (mcEq/100g) | Pepsin output (mg of tyrosine/100g) |
|---|---|---|---|---|---|
| Control | 4.04 ± 0.24 | 1.09 ± 0.03 | 345.0 ± 32.8 | 387.1 ± 33.4 | 39.9 ± 2.7 |
| Ex. 1 | 1.12 ± 0.18** | 2.02 ± 0.31* | 33.9 ± 14.9** | 70.6 ± 17.4** | 12.0 ± 1.6** |
| Ex. 2 | 1.63 ± 0.28** | 1.29 ± 0.08* | 111.0 ± 36.3** | 147.2 ± 37.4** | 15.9 ± 2.1** |
| Ex. 5 | 1.41 ± 0.15** | 1.48 ± 0.11** | 68.4 ± 19.6** | 117.0 ± 20.8** | 16.6 ± 2.2** |
| Ex. 6 | 2.00 ± 0.27** | 1.36 ± 0.17 | 132.7 ± 33.8** | 172.6 ± 36.5** | 21.6 ± 2.7** |
| Ex. 7 | 2.91 ± 0.29** | 1.13 ± 0.03 | 220.6 ± 32.5* | 260.5 ± 32.9* | 27.2 ± 2.3** |

* $P < 0.05$ ** $P < 0.01$

**Table 6**

| Test compound | Gastric juice (mℓ) | Gastric juice (pH) | Free HCℓ (mEq/1) | Total acidity (mEq/1) | Pepsin output (units/hr) |
|---|---|---|---|---|---|
| Control | 5.9 ± 0.5 | 1.47 ± 0.03 | 47.6 ± 3.2 | 71.3 ± 3.6 | 153.8 ± 12.8 |
| Ex. 3 | 6.4 ± 0.7 | 1.66 ± 0.09 | 35.1 ± 5.0* | 66.6 ± 4.7 | 157.7 ± 17.6 |
| Control | 6.0 ± 0.4 | 1.41 ± 0.04 | 55.4 ± 3.7 | 84.9 ± 4.3 | 144.9 ± 10.6 |
| Ex. 4 | 3.6 ± 0.8** | 2.54 ± 0.27** | 14.3 ± 7.4*** | 68.8 ± 3.6* | 83.1 ± 19.4** |

* $P < 0.05$  ** $P < 0.01$  *** $P < 0.001$

## EXPERIMENT 5

### Acute Toxicity

A suspension of each test compound in 5% gum arabic aqueous solution was orally given to SLC-ICR

or SLC-ddy strain male mice (at the age of 6 to 7 weeks, weighing 26.6 to 33.6 g; 3-8 mice in one group) forcedly once at a dose of 500 mg and 1,000mg of the test compound/5mℓ/kg. For 7 days after the administration, the mice were observed whether they were dead or alive.

The results are shown in Table 7.

Table 7

| Test compound | Mortality rate (No. of death/No. of treated) | |
|---|---|---|
| | 500 mg/kg | 1000 mg/kg |
| Ex. 1 | 0/5 | 1/5 |
| Ex. 2 | 0/5 | 0/5 |
| Ex. 3 | 2/6 | 3/3 |
| Ex. 4 | 3/8 | 5/5 |
| Ex. 5 | 2/5 | 3/5 |
| Ex. 6 | 0/5 | 0/5 |
| Ex. 7 | 0/5 | 0/5 |

The compounds of the present invention possess a potent acid secretion inhibiting activity and exhibit potent antiulcer activity against aspirin-induced ulcer and water-immersion stress ulcer, and ethanol-induced ulcer. Further, the compounds of the present invention are low-toxic so that they are also useful as medical agents for man.

While the present invention has been described in detail and with reference to specific embodiments thereof, it is apparent to one skilled in the art that various changes and modifications can be made therein without departing from the spirit and the scope of the present invention.

## Claims

1. An imidazo[2,1-b]thiazole compound represented by formula (I) and pharmacologically acceptable salts and optical isomers thereof:

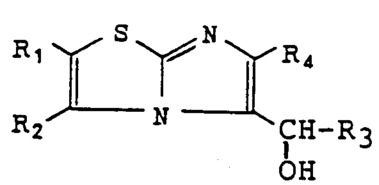

(I)

wherein $R_1$ and $R_2$ may be the same or different groups and each represents a hydrogen atom or a lower alkyl group having 1 to 6 carbon atoms; $R_3$ represents a hydrogen atom, a lower alkyl group having 1 to 6 carbon atoms, a phenyl group or a phenyl group substituted with methyl group(s), methoxy group(s) or halogen atom(s); and $R_4$ represents a hydrogen atom or a lower alkyl group having 1 to 3 carbon atoms, with the proviso that when $R_3$ represents a hydrogen atom, at least one of $R_1$ and $R_2$ represents a lower alkyl group having 1 to 6 carbon atoms.

**2.** The compound and pharmacologically acceptable salts and optical isomers thereof according to claim 1, wherein $R_4$ is a methyl group.

**3.** The compound and pharmacologically acceptable salts and optical isomers thereof according to claim 1, wherein $R_1$ and $R_2$ are the same or different groups and each is a hydrogen atom or a lower alkyl group having 1 to 4 carbon atoms.

**4.** The compound and pharmacologically acceptable salts thereof according to claim 1, wherein $R_3$ is a hydrogen atom or wherein $R_3$ is a lower alkyl group having 1 to 4 carbon atoms including the optical isomers thereof.

**5.** The compound and pharmacologically acceptable salts and optical isomers thereof according to claim 1, wherein $R_3$ is a phenyl group.

**6.** The compound and pharmacologically acceptable salts thereof according to claim 1, wherein $R_2$ is a lower alkyl group having 1 to 6 carbon atoms, and $R_3$ is a hydrogen atom.

**7.** 3,6-Dimethyl-5-hydroxymethylimidazo[2,1-b]thiazole and pharmacologically acceptable salts thereof.

**8.** 5-(hydroxy-phenyl)methyl-6-methylimidazo[2,1-b] thiazole and pharmacologically acceptable salts and optical isomers thereof.

**9.** 5-(1-hydroxybutyl)-6-methylimidazo[2,1-b]thiazole and pharmacologically acceptable salts and optical isomers thereof.

**10.** Use of the compounds according to any of claims 1 to 9 and their pharmacologically acceptable salts for the preparation of pharmaceutical compositions having gastric acid inhibiting activity.

**11.** An antiulcer agent comprising, as the active ingredient, the compound or pharmacologically acceptable salt thereof according to any of claims 1 to 9.

**12.** A process for the production of compounds I according to claim 1 characterized by reacting an 2-aminothiazole II

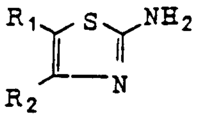

(II)

wherein $R_1$ and $R_2$ are as defined in claim 1,

(a) for preparing compounds I wherein $R_4$ is methyl, with a propargyl/halide to obtain a 2-imino 3-propargylthiazole (II-a)

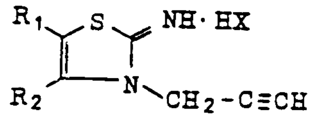

(II-a)

wherein $R_1$ and $R_2$ are as defined above, and X represents a halogen, cyclizing the resulting product to obtain a compound (I-a)

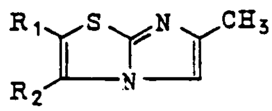

( I − a )

wherein $R_1$ and $R_2$ are as defined above, and then introducing the required -CH(OH)$R_3$ group at the 5-position in a manner known per se, or

(b) for preparing compounds I wherein $R_4$ is as defined in claim 1, with a halogenated ketone compound to obtain the cyclized compound (I-b)

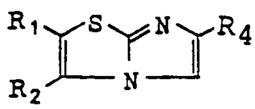

( I − b )

wherein $R_1$, $R_2$ and $R_4$ are as defined in claim 1, and then introducing the required -CH(OH)-$R_3$ group into the 5-position in a manner known per se;

and optionally converting the compounds into their pharmacologically acceptable salts in a manner known per se;

and/or optionally separating the compounds into their optically active isomers in a manner known per se.

**Claims for the following Contracting State: ES**

**1.** A process for preparing an imidazo [2,1-b] thiazole compound represented by formula (I) and pharmacologically acceptable salts and optical isomers thereof:

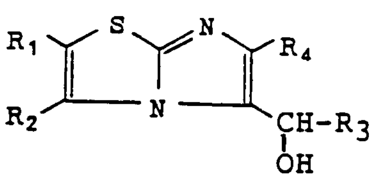

(I)

wherein $R_1$ and $R_2$ may be the same or different groups and each represents a hydrogen atom or a lower alkyl group having 1 to 6 carbon atoms; $R_3$ represents a hydrogen atom, a lower alkyl group having 1 to 6 carbon atoms, a phenyl group or a phenyl group substituted with methyl group(s), methoxy group(s) or halogen atom(s); and $R_4$ represents a hydrogen atom or a lower alkyl group having 1 to 3 carbon atoms, with the proviso that when $R_3$ represents a hydrogen atom, at least one of $R_1$ and $R_2$ represents a lower alkyl group having 1 to 6 carbon atoms, characterized by reacting an 2-aminothiazole II

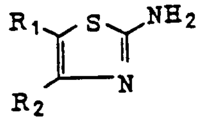

( II )

wherein $R_1$ and $R_2$ are as defined in claim 1,

(a) for preparing compounds I wherein $R_4$ is methyl, with a propargyl/halide to obtain a 2-imino 3-propargylthiazole (II-a)

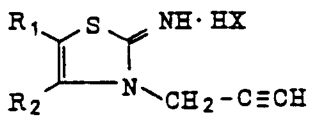

(II-a)

wherein $R_1$ and $R_2$ are as defined above, and X represents a halogen, cyclizing the resulting product to obtain a compound (I-a)

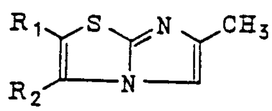

(I-a)

wherein $R_1$ and $R_2$ are as defined above, and then introducing the required -CH(OH)$R_3$ group at the 5-position in a manner known per se, or

(b) for preparing compounds I wherein $R_4$ is hydrogen or lower alkyl with 1 to 3 carbon atoms, with a halogenated ketone compound to obtain the cyclized compound (I-b)

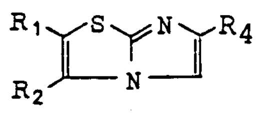

(I-b)

wherein $R_1$, $R_2$ and $R_4$ are as defined in claim 1, and then introducing the required -CH(OH)-$R_3$ group into the 5-position in a manner known per se; and optionally converting the compounds into their pharmacologically acceptable salts in a manner known per se; and/or optionally separating the compounds into their optically active isomers in a manner known per se.

**2.** The process according to claim 1, wherein $R_4$ is a methyl group.

**3.** The process according to claim 1, wherein $R_1$ and $R_2$ are the same or different groups and each is a hydrogen atom or a lower alkyl group having 1 to 4 carbon atoms.

**4.** The process according to claim 1, wherein $R_3$ is a hydrogen atom or a lower alkyl group having 1 to 4 carbon atoms.

**5.** The process according to claim 1, wherein $R_3$ is a phenyl group.

**6.** The process thereof according to claim 1, wherein $R_2$ is a lower alkyl group having 1 to 6 carbon atoms, and $R_3$ is a hydrogen atom.

**7.** The process according to claim 1 for preparing 3,6-Dimethyl-5-hydroxymethylimidazo[2,1-b]thiazole and pharmacologically acceptable salts thereof.

**8.** The process according to claim 1 for preparing 5-(hydroxy-phenyl)methyl-6-methylimidazo[2,1-b]-thiazole and pharmacologically acceptable salts and optical isomers thereof.

9. The process according to claim 1 for preparing 5-(1-hydroxybutyl)-6-methylimidazo[2,1-b]thiazole and pharmacologically acceptable salts and optical isomers thereof.

European Patent Office

# EUROPEAN SEARCH REPORT

Application Number

EP 90 11 2254

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF HETEROCYCLIC CHEMISTRY, vol. 26, no. 2, March 1989, pages 525-529, Heterocorp., Tampa, FL, US; P. CARLONI et al.: "Imidazo[2,1-b]thiazole carbamates and acylureas as potential insect control agents" * Page 525, structure 4f * --- | 1 | C 07 D 513/04<br>A 61 K 31/425 //<br>(C 07 D 513/04<br>C 07 D 277:00<br>C 07 D 235:00 ) |
| A | EP-A-0 347 880 (NIKKEN CHEMICALS) * Claims 1,13 * --- | 1,11 | |
| A | PATENT ABSTRACTS OF JAPAN, vol. 14, no. 118 (C-697)[4061], 6th March 1990; & JP-A-1 319 488 (NIKKEN CHEMICALS) 25-12-1989 * Abstract * --- | 1,11 | |
| A | FR-A-2 260 344 (PLANTEX) * Claims * ----- | 1 | |
| | | | TECHNICAL FIELDS SEARCHED (Int. Cl.5) |
| | | | C 07 D 513/00<br>A 61 K 31/00 |

The present search report has been drawn up for all claims

| Place of search | Date of completion of the search | Examiner |
|---|---|---|
| THE HAGUE | 19-02-1991 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)